# EUROPEAN PATENT SPECIFICATION

(11) **EP 1 436 282 B1**
(45) Date of publication and mention of the grant of the patent: **22.06.2005**
(21) Application number: 02793797.8
(22) Date of filing: 15.10.2002
(51) Int. Cl.: C07D 401/06

(54) **SYNTHESIS OF 4-(PIPERIDYL) (2-PYRIDYL)METHANONE-(E)-O-METHYLOXIME AND ITS SALTS**
SYNTHESE VON 4-(PIPERIDYL) (2-PYRIDYL)METHANON-(E)-O-METHYLOXIM UND SEINEN SALZEN
SYNTHESE DE 4-(PIPERIDYL) (2-PYRIDYL)METHANONE-(E)-O-METHYLOXIME ET SES SELS

(30) Priority: 15.10.2001 US 329561 P
(43) Date of publication of application: 14.07.2004
(73) Proprietor: SCHERING CORPORATION, Kenilworth, New Jersey 07033-0530 (US)
(72) Inventor: WU, Wenxue, Princeton Junction, NJ 08550 (US); LIAO, Hongbiao, Bridgewater New Jersey 08807 (US); TSAI, David, J., S., Warren, NJ 07059 (US)
(74) Representative: Gross, Ulrich-Maria
(86) International application number: PCT/US2002/033118
(87) International publication number: WO 2003/033488

(56) References cited:
- WO-A-02/32893
- PALANI A ET AL: "Discovery of 4-[(Z)-(4-Bromophenyl)-(ethoxyimino)methyl ]-1'-[(2,4-dim ethyl-3-pyridinyl)carbonyl]-4'-methyl-1,4' -bipiperidine N-Oxide (SCH 351125): An Orally Bioavailable Human CCR5 Antagonist for the Treatment of HIV Infection" JOURNAL OF MEDICINAL CHEMISTRY, AMERICAN CHEMICAL SOCIETY. WASHINGTON, US, vol. 44, no. 21, 11 October 2001 (2001-10-11), pages 3339-3342, XP002220286 ISSN: 0022-2623
- PIWINSKI J J ET AL: "Dual antagonists of platelet activating factor and histamine.Identification of structural requirements for dual activity of N-acyl-4-(5,6-dihydro-11H-benzo[5,6]cycloh epta-[1,2-b]pyridin-11-y lidene)piperidines" JOURNAL OF MEDICINAL CHEMISTRY, AMERICAN CHEMICAL SOCIETY. WASHINGTON, US, vol. 34, no. 1, 1991, pages 457-461, XP002169640 ISSN: 0022-2623

## Description

This application specifically discloses a novel process to synthesize 4-(piperidyl) (2-pyridyl)methanone-(E)-O-methyloxime and its salts in high stereochemical purity. It also generically discloses a process to prepare compounds similar to the above in high stereochemical purity. This application claims priority from U.S. provisional application, Serial No. 60/329,561 filed on October 15, 2001.The invention disclosed herein is related to that disclosed in the provisional patent application, Serial Number 60/329,562 filed on October 15, 2001 (WO 02/32893).

### Background of the Invention

4-(Piperidyl) (2-pyridyl)methanone-(E)-O-methyloxime dihydrochloride (Formula I) is an intermediate used in the preparation of compounds that are histamine-H₃ antagonists. An example of such histamine-H₃ antagonists is 1-[[1-[(2-Amino-5-pyrimidinyl)methyl]-4-piperidinyl]carbonyl]-4-[(E)-(methoxyimino)-2-pyridinylmethyl]piperidine shown in Formula II.

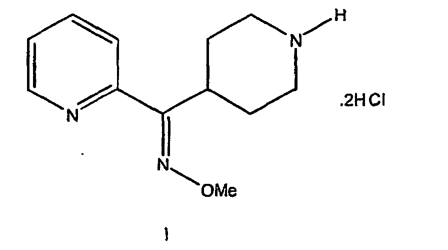

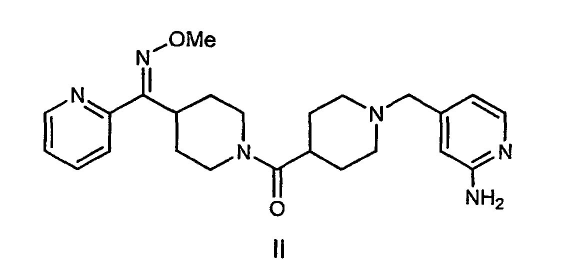

The conversion of the compound of Formula I into a compound of Formula II is disclosed in the commonly owned U.S. patent application, Serial No. 09/978,267 (Attorney Docket No. AL01348K) filed of even date herewith. Antagonists of the H₃ receptor are useful for the treatment of allergy, asthma and other such respiratory disorders.

The preparation of oximes and their isomerization is known from Palani et al., J. Med. Chem 2001, 44 (21), 3339-3342.

In view of the importance of the antagonists of histamine-H₃, new, novel methods of making such antagonists and/or their intermediates are always of interest.

### Summary of the Invention

In an embodiment, the present application teaches a novel, simple process of making a compound of Formula I, its monohydrochloride and its free base itself in high stereochemical purity and, via that process, a method of making a compound of Formula II in high yields and high stereochemical purity. The term "high stereochemical purity" refers to at least about 90% of the desired isomer, which, in the present invention, is the E-isomer of the compound of Formula I, its monohydrochloride and its free base. Indeed, the stereochemical purity of the compound of Formula I, its monohydrochloride and its free base made by the inventive process typically exceeds 95% of the E-isomer. The term "high yields" refers to at least about 60% yield of the desired product.

Thus, the present process comprises synthesizing compounds such as the compound of Formula I, its mono acid salt (for example, its monohydrochloride) and its free base from a compound of Formula III: 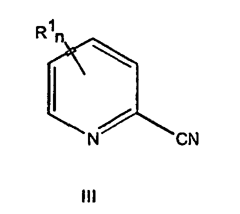 where R¹ is defined below and n is a number from 1 to 4, and from a compound of Formula IV: 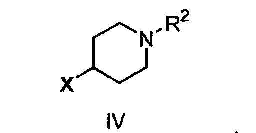 where R² is defined below. The process of making a compound such as the compound of Formula I from a compound of Formula III and a compound of Formula IV comprises:
(a) converting the compound of Formula IV into its Grignard form of Formula IVA: 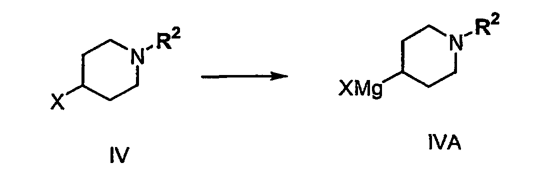 where R² is defined below and X is a halogen;
(b) reacting the compound of Formula III with the compound of Formula IVA to obtain a compound of Formula V: 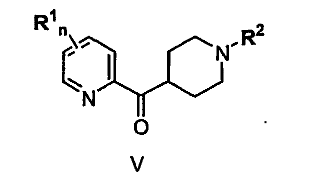
(c) reacting the compound of Formula V with a suitable alkyl chloroformate of Formula VI:

   R³-OCOCl VI

   where R³ is defined below, to yield a compound of Formula VII: 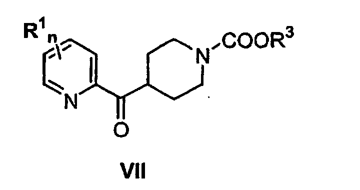
(d) forming the free base (Formula VIIA) and then the acid salt (mono acid salt or diacid salt) of the free base (Formula VIII): 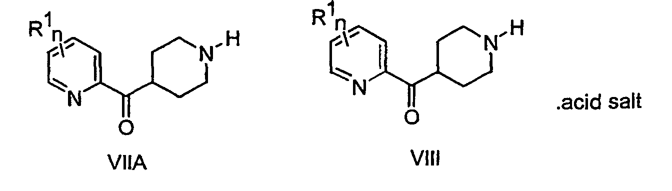
(e) reacting the compound of Formula VIII with an alkoxyamine (NH₂OR⁴) or its hydrochloride (where R⁴ is defined below) to form an oxime of Formula IX: 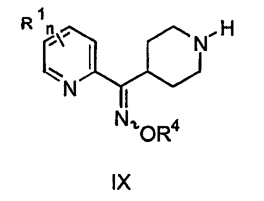 and
(f) isomerizing the compound of Formula IX predominantly to the E isomer by treatment with a strong acid and simultaneously converting to the desired acid salt of a compound such as the compound of Formula I with an enriched E isomer, wherein the E isomer predominates over the Z-isomer by at least a 90:10 ratio. The acid salt, which may be the mono acid salt or the diacid salt, may be optionally converted back to its free base, if so desired.

R¹, R², R³ and R⁴ may be the same or different and are independently selected from the group consisting of H, halogen, alkyl, aryl, alkoxy, aryloxy, aralkyl (with the alkyl being the linker), alkylaryl (with the aryl being the linker), heteroalkyl, heteroaryl, alkyl-heteroaryl, heteroaralkyl, cycloalkyl and cycloalkylalkyl, wherein said alkyl, aryl, alkoxy, aryloxy, arylalkyl, alkylaryl, heteroalkyl, heteroaryl, alkyl-heteroaryl, heteroaralkyl, cycloalkyl and cycloalkylalkyl may optionally be substituted with one or more chemically-suitable substituents independently selected from the group consisting of alkyl, alkenyl, alkynyl, aryl, aralkyl, cycloalkyl, heterocyclic and halogen. R¹ itself may be F, Cl, Br or I. The term "halogen" refers to F, Cl, Br or I. The acid-catalyzed isomerization in step (f) above is believed to be novel and offers the desired salt of the desired compound with the enriched E-isomer as noted above. When R¹ is H, n=1, R⁴ = methyl, and the acid used in step (f) for isomerization is HCl in the above sequence, the final product is the compound of Formula I.

The inventive process to make the compound of Formulas IX and I has several advantages: it is economical, can be easily scaled-up and yields the desired E-isomer in high yields and in high stereochemical purity.

### Description of the Invention

In one embodiment, the present invention discloses a novel, easy-to-use process for preparing the compound such as the compound of Formula I in high yields and high stereochemical purity. Additionally, it teaches novel processes to prepare intermediates such as the compounds of Formulas V, VII, VIII and IX in high yields. The inventive process to prepare such compounds is schematically described below in Scheme 1: 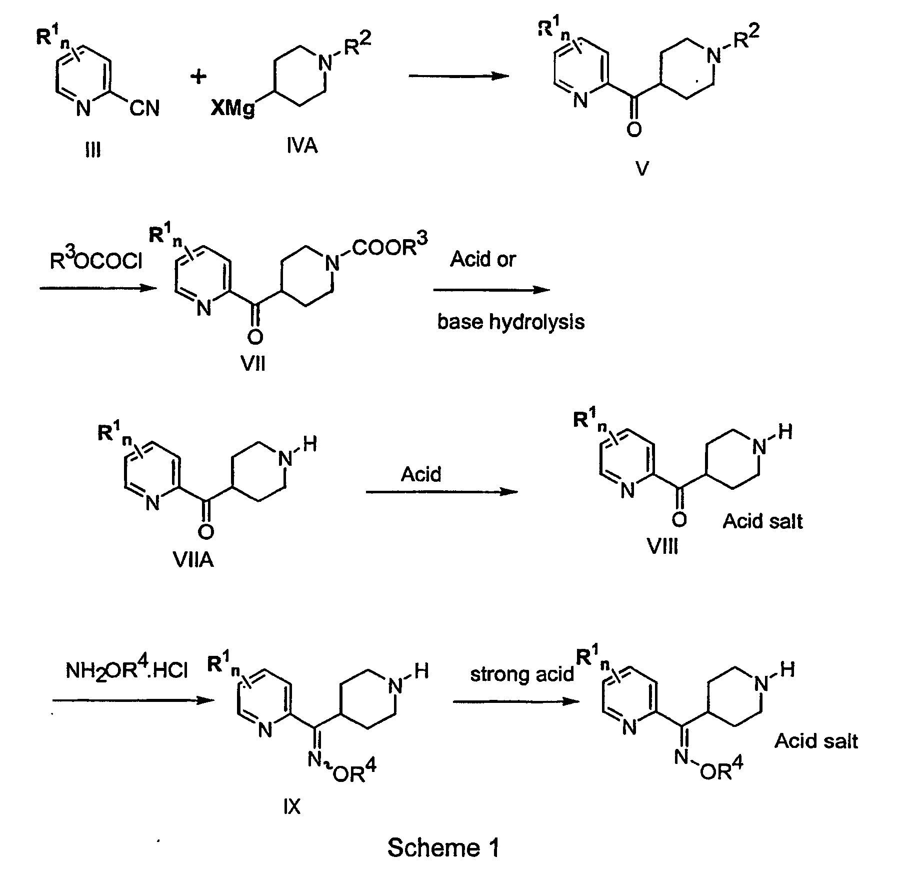 where the various terms are defined above.

Unless defined otherwise, all technical and scientific terms used herein have the same meaning as is commonly understood by one of skill in the art to which this invention belongs. Thus, for example, the term alkyl (including the alkyl portions of alkoxy) refers to a monovalent group derived from a straight or branched chain saturated hydrocarbon by the removal of a single atom having from 1 to 8 carbon atoms, preferably from 1 to 6;
aryl - represents a carbocyclic group having from 6 to 14 carbon atoms and having at least one benzenoid ring, with all available substitutable aromatic carbon atoms of the carbocyclic group being intended as possible points of attachment. Preferred aryl groups include phenyl, 1-naphthyl, 2-naphthyl and indanyl, and especially phenyl and substituted phenyl;
aralkyl - represents a moiety containing an aryl group linked vial a lower alkyl;
alkylaryl - represents a moiety containing a lower alkyl linked via an aryl group;
cycloalkyl - represents a saturated carbocyclic ring having from 3 to 8 carbon atoms, preferably 5 or 6, optionally substituted.
halogen - represents fluorine, chlorine, bromine and iodine; preferred halogens are Cl and Br.
heteroaryl - represents a cyclic organic group having at least one O, S and/or N atom interrupting a carbocyclic ring structure and having a sufficient number of delocalized pi electrons to provide aromatic character, with the aromatic heterocyclic group having from 2 to 14, preferably 4 or 5 carbon atoms, e.g., 2-, 3- or 4-pyridyl, 2- or 3-furyl, 2- or 3-thienyl, 2-, 4- or 5-thiazolyl, 2- or 4-imidazolyl, 2-, 4- or 5-pyrimidinyl, 2-pyrazinyl, or 3- or 4-pyridazinyl, etc. Preferred heteroaryl groups are 2-, 3- and 4-pyridyl; Such heteroaryl groups may also be optionally substituted.
heteroalkyl- represents an alkyl group containing one or more heteroatoms.

The synthesis of the specific compound of Formula I, following the above-noted process, is exemplified in Scheme 2: 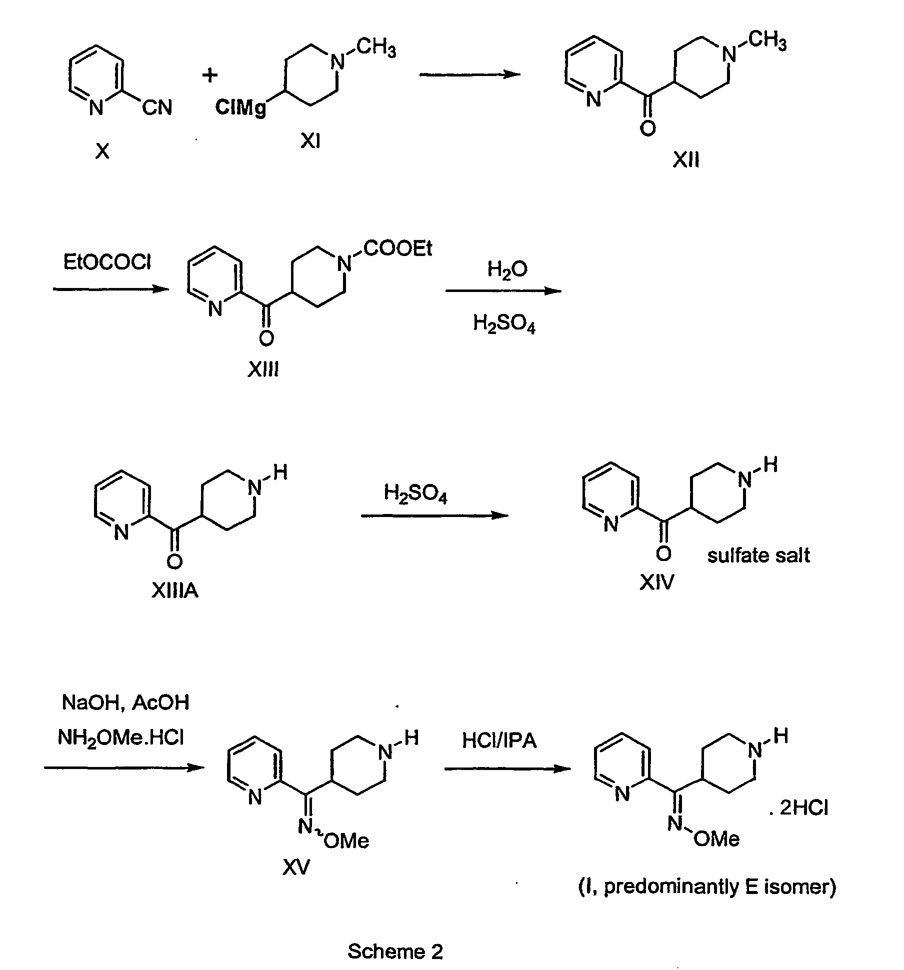 The compounds of the Formulas XII, XIII, XIIIA, XIV and XV and their isomers (where applicable) are believed to be novel compounds. As stated above, the inventive novel conversion of the compound of Formula XV to I surprisingly yields predominantly the E-isomer of the compound of Formula I in high stereochemical purity and high yields. Isomerization of a mixture of phenyl compounds by acid catalysis is discussed by T. Zsuzsanna *et al, Hung.Magy.Km.Foly.,* 74(3) (1968), 116-119. While the preferred reagents and reaction conditions for the various steps in the inventive process are described in detail in the **Examples** section, the following summarizes the details for the generic synthesis according to Scheme 1.

The presently disclosed process starts with the compound of Formula IV. In step 1, a 4-halo-1-R² substituted piperidine is converted to its Grignard analog (IV) by reacting with magnesium. The reaction is performed generally at temperatures of about -10° C to reflux. Generally a hydrocarbon solvent such as, for example, toluene, xylene, chlorobenzene, and the like, an ether such as, for example, a C₅-C₁₂ alkyl ether, 1,2-dimethoxyethane, 1.2-diethoxyethane, diglyme, 1,4-dioxane, tetrahydrofuran, methyl tetrahydrofuran, and the like, or a mixture of such solvents, is suitable for this reaction. The solution is cooled to around -10° C to about 10° C and then reacted with a suitable 2-cyanopyridine (III), for about 10-120 minutes. Examples of suitable 2-cyanopyridine are 2-cyanopyridine, 4-methyl-2-cyanopyridine, 4-ethyl-2-cyanopyridine, 4-phenyl-2-cyanopyridine, and the like. Preferred are 2-cyanopyridine and 4-methyl-2-cyanopyridine. Compounds such as, for example, Red-Al® (from Aldrich Chemical Company, Milwaukee, Wisconsin), iodine and the like, may be used as initiators in this reaction. The Grignard compound is used generally in about 1-4 molar equivalents with respect to the compound of formula III, preferably in about 1-3 molar equivalents and typically in about 1.5-2.5 molar equivalents. The product of formula V may be isolated by customary work-up procedures, such as, for example, treatment with an acid (e.g. HCl) preferably in a suitable solvent (e.g., tetrahydrofuran or ethyl acetate).

The product of Formula V may then be reacted with an alkyl chloroformate in the next step. Suitable alkyl chloroformates are, for example, methyl chloroformate, ethyl chloroformate, propyl chloroformate, benzyl chloroformate., and the like, with the preferred being methyl chloroformate or ethyl chloroformate. Generally a solvent such as, for example, toluene, xylene, chlorobenzene, methylene chloride, ethylene chloride, ethyl acetate, isobutyl acetate, n-butyl acetate, a C₅-C₁₂ alkyl ether, 1,2-dimethoxyethane, 1.2-diethoxyethane, diglyme, 1,4-dioxane, tetrahydrofuran, methyl tetrahydrofuran and the like is suitable for this reaction. The reaction is generally performed at about 25-100°C, preferably about 40-90°C and typically about 50-80°C, for about 1-5 hours. After the reaction, generally the generated acid is washed off and the product of formula VII may be isolated by organic solvent extraction.

The compound of Formula VII may then be hydrolyzed to its free base (Formula VIIA) by acid (or base) hydrolysis, which may then be converted into its acid salt (Formula VIII) by treatment with an acid such as, for example, sulfuric acid, hydrochloric acid, trifluoroacetic acid and the like, generally in a solvent at temperatures between ambient and reflux of the solvent. Suitable solvent is water containing the acid whose salt is desired. The salt may be recrystallized. Suitable recrystallization solvents include water, water-miscible solvents such as, for example, acetonitrile, THF, ethanol, methanol, acetone and the like, and mixtures thereof; acetonitrile or acetonitrile-water mixture is preferred. There being two nitrogen atoms in the compound of Formula VIIA, the salt VIII may have 1 or 2 moles of acid.

The compound of Formula VIII may then be converted to an alkyloxime of Formula IX by reacting it with an alkoxyamine (or its hydrochloride), usually in a protic solvent; water is preferred. Suitable alkoxyamines are, for example, methoxyamine, ethoxyamine and the like. Methoxyamine is preferred. The alkoxyamine (or its hydrochloride) is employed generally in about 1 to about 4 molar equivalents, preferably in about 1 to about 3 molar equivalents, and typically in about 1 to about 2 molar equivalents, with respect to the compound of Formula VIII. Generally, the reaction is catalyzed by a weak acid such as, for example, acetic acid, formic acid and the like, or mixtures thereof. The pH may be adjusted to be about 3-6 if so desired. A cosolvent such as, for example, methanol, ethanol, isopropanol, n-butanol and the like, or mixtures thereof may be added, if so desired. The product of Formula IX, after work-up, is a mixture of the Z- and the E-isomers, whose ratio may be analyzed for its stereochemical make-up, using techniques well known in the art such as, for example, HPLC.

Since the desired isomer is the E-isomer, it would be advantageous to enrich the compound of Formula IX in the desired E-isomer. Applicants found that treating the compound of Formula IX with a strong acid under certain reaction conditions surprisingly isomerizes the mixture of the Z and the E-isomers into predominantly the E-isomer. Generally, the compound of Formula IX may be dissolved in a solvent such as, for example, ethanol, methanol, isopropanol, n-butanol and the like, ether such as methyl tart-butyl ether, tetrahydrofuran and the like, hydrocarbon such as, for example, heptane, hexane, toluene and the like, nitrile such as, for example, acetonitrile and the like, or mixtures of such solvents. It is then treated with a strong acid such as, for example, HCl, HBr, H₂SO₄ and the like, at temperatures in the range 20 to 100°C for about 1-20 hours. The acid is employed generally in about 1 to about 10 molar equivalents, preferably in about 1 to about 8 molar equivalents, and typically in about 1 to about 6 molar equivalents. Work-up typically forms predominantly the acid salt of the E-isomer of the compound of Formula IX. Depending upon the reaction conditions, there may be one (e.g. 1HCl), or two (e.g. 2HCl) molar equivalents of the acid in the isolated E isomer, since the compound contains two nitrogen atoms. As one skilled in the art knows, the final product may optionally be converted to its free base with the E isomer still predominating, by reacting with standard processes such as, for example, treatment with a suitable base.

When R²= R³=R⁴= methyl, n=1 and R¹= H, and the acid salt is 2HCl in the isolated E isomer compound, it is in fact the compound of Formula I. HPLC analysis (when R²= R³=R⁴= methyl, n=1 and R¹= H and the acid salt is 2HCl) after a typical reaction sequence as shown in the **Examples** section showed the presence of the E-isomer generally in about 90% or above stereochemical purity, and typically in about 95% or above stereochemical purity in the isolated product. Additionally, the yields of the desired compound in such stereochemical purity was quite high, demonstrating that such isomerization reaction using a strong acid may be applicable to prepare E-isomers of such oximes in high yields and high stereochemical purity.

The products of the various steps in the reaction schemes described herein may be isolated and purified by conventional techniques such as, for example, filtration, recrystallization, solvent extraction, distillation, precipitation, sublimation, column chromatography and the like, as is well known to those skilled in the art. The products may be analyzed and/or checked for purity by conventional methods such as, for example, thin layer chromatography, NMR, HPLC, melting point, mass spectral analysis, elemental analysis and the like, well known to those skilled in the art.

The following nonlimiting EXAMPLES are provided in order to further illustrate the present invention. While the EXAMPLES are described herein as the preparation of the compound of Formula I from the compound of Formula X as shown in Scheme 2, it will be apparent to those skilled in the art that many modifications, variations and alterations to the present disclosure, both to materials, methods and reaction conditions, may be practiced. All such modifications, variations and alterations are intended to be within the spirit and scope of the present invention.

### EXAMPLES

Unless otherwise stated, the following abbreviations have the stated meanings in the Examples below:
HPLC= High Performance Liquid Chromatography
M.pt: melting point
NMR= nuclear magnetic resonance spectroscopy
DMSO= dimethylsulfoxide
mL= milliliters
g= grams
rt= room temperature (ambient)

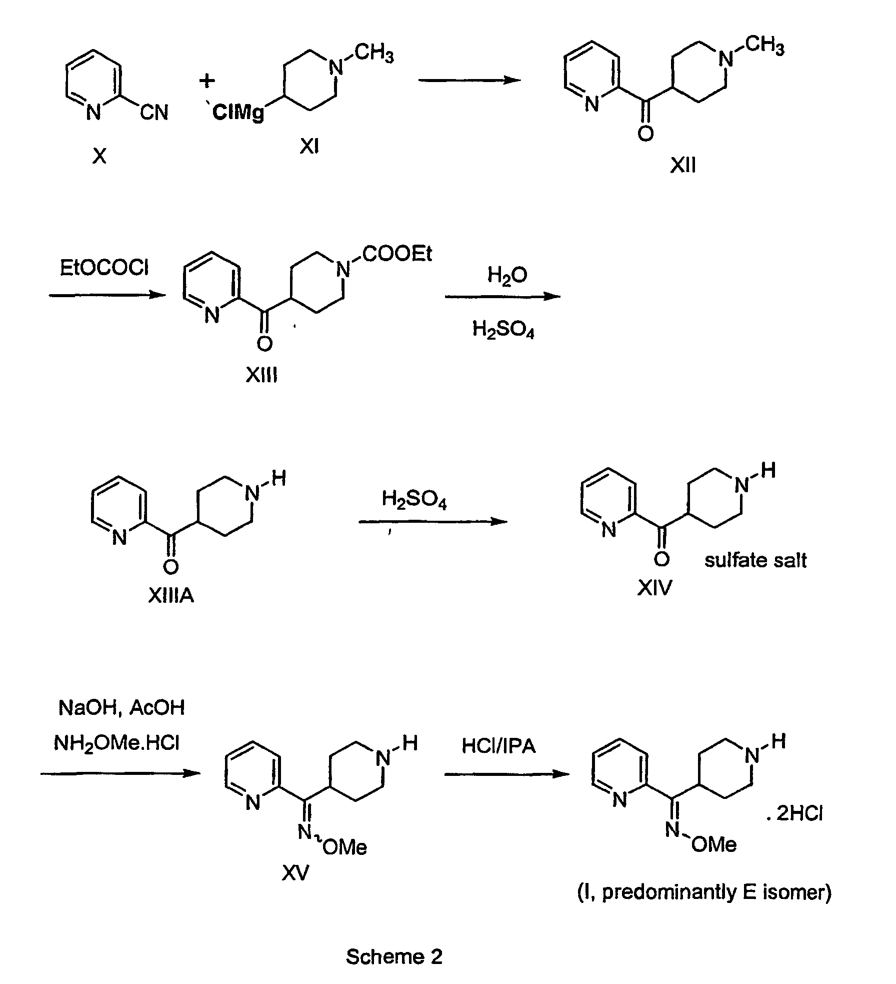

**Example 1. Preparation of the Compound of Formula XII:**To a suspension of magnesium chips (110 g) in THF (2800 mL) was added Red-Al® (9 mL, 65% solution of sodium bis(2-methoxyethoxy)aluminum hydride in toluene). The mixture was heated at reflux for 1 h and then cooled to room temperature. 4-chloro-1-methylpiperidine (71 mL) was added and the mixture was heated at gentle reflux for 30 min or until the Grignard reaction was initiated. The main portion of 4-chloro-1-methylpiperidine (633 mL) was then added over 60 min while maintaining the reaction mixture at gentle reflux. After the addition was complete, the mixture was heated at reflux for 5 h and then cooled to -5 to 0°C. A solution of 2-cyanopyridine (281 g, from Aldrich Chemical Company) in THF (560 mL) was added over 1 h at -5 to 5°C. The mixture was stirred at -5 to 5°C for 30 min and poured into a mixture of concentrated hydrochloric acid (600 mL) and ice (3000 g). The phases were separated. To the aqueous layer was added sodium chloride (600 g) and the resulting solution was extracted with THF (2200 mL) three times. The organic layers were combined and concentrated under vacuum to give a brown oil (501 g). The oil was found to be 86.1 % pure by HPLC analysis against a pure standard. The crude material could be used directly in the next step or purified, if so desired. The crude product was purified by vacuum distillation to give a yellow oil which solidified upon cooling (b.p.: 120-125°C/0.5 torr, low melting solid). ¹H NMR (400 MHz, CDCl₃): δ 8.42 (dd, J₁=3.3 Hz, J₂=0.9 Hz 1H), 7.76 (d, J=7.8 Hz, 1 H), 7.58 (dt, J₁=7.7 Hz, J₂=1.7 Hz, 1H), 7.21 (ddd, J₁=7.5 Hz, J₂=4.8 Hz, J₃=1.2 Hz, 1H), 3.56 (tt, J₁=11.5 Hz, J₂=3.8 Hz, 1H), 2.65 (m, 2H), 2.03 (s, 3H), 1.85 (dt, J₁=11.7 Hz, J₂=2.5 Hz, 2H), 1.67 (br d, J=12.4 Hz, 2H), 1.53 (m, 2H).

**Example 2. Preparation of the Compound of Formula XIII:**A sample of crude compound of Formula XII (from Example 1) (249 g, 60.4% purity) was azeotropically dried in toluene. To the dried solution in toluene (2000 mL) was added ethyl chloroformate (169 mL) over 30 min at 70-75°C. The reaction mixture was heated at 70-80 °C for 2 h and cooled to room temperature. An aqueous potassium bicarbonate solution (300 ml, 25%) was added over 30 min at 20 to 30°C. After stirring at room temperature for 15 min, the mixture was settled and the phases were separated. The organic layer was washed with 10% aqueous acetic acid (1000 mL) followed by water (1000 mL). The organic layer thus obtained (2720 mL) was found to contain 170 g of the compound of Formula XIII by HPLC analysis against a pure standard. The toluene solution can be used directly for the preparation of the compound of Formula XIV.

An analytically pure sample of the compound of Formula XIII was obtained by flash column chromatography (pale yellow solid, m.p. 54.4°C). ¹H NMR (400 MHz, CDCl₃): δ 8.70 (dd, J₁=5.3 Hz, J₂=0.9 Hz, 1H), 8.05 (d, J=7.8 Hz, 1H), 7.86 (dt, J₁=7.7 Hz, J₂=1.7 Hz, 1H), 7.50 (m, 1H), 4.23 (br s, 2H), 4.15 (q, J=7.1 Hz, 2H), 4.05 (tt, J₁=11.5 Hz, J₂=3.9 Hz, 1H), 2.99 (br t, J=11.6, 2H), 1.91 (br s, 2H), 1.65 (dq, J₁=12.2 Hz, J₂=3.6 Hz, 2H), 1.28 (t, J=7.1 Hz, 3H).

**Example 3. Preparation of the Compound of Formula XIIIA and conversion into the Compound of Formula XIV:** The above toluene solution (from Example 2) was extracted into 50% v/v sulfuric acid (330 mL) and the acid layer was heated at 90-100°C for 20 h. The mixture was cooled to 50-60 °C and diluted with acetonitrile (2000 mL) and seeded. The mixture was cooled to room temperature and was filtered. The wet product was washed with acetonitrile and dried at 55-65°C under vacuum (248 g, brown solid).

**Example 4. Preparation of the Compound of Formula XIV from the Compound of Formula XII:** A sample of crude compound of Formula XII (240 g, 86.1% purity) was azeotropically dried in toluene. To the dried solution in toluene (2000 mL) was added ethyl chloroformate (169 mL) over 30 min at 70-75°C. The reaction mixture was heated at 70-80°C for 5 h, over which time, triethylamine (21 mL) and more ethyl chloroformate (22 mL) were added. An aqueous potassium bicarbonate solution (300 ml, 25%) was added over 30 min at 20 to 30°C. After stirring at room temperature for 15 min, the mixture was settled and the phases were separated. The organic layer was washed with 10% aqueous acetic acid (1000 mL) followed by water (1000 mL). The organic layer was extracted into 50% v/v sulfuric acid (450 mL) and the acid layer was heated at 90-100°C for 16 h. The mixture was cooled to 50-60°C and diluted with acetonitrile (2000 mL) and seeded. The mixture was cooled to room temperature and was filtered. The wet product was washed with acetonitrile and dried at 55-65°C under vacuum (360 g, off-white solid, m.p.: 247°C dec.). ¹H NMR (400 MHz, DMSO-d₆): 10.68 (br s, 3H), 8.76 (m, 1H), 8.63 (br s, 1H), 8.33 (br s, 1H), 8.03 (m, 2H), 7.72 (ddd, J₁=7.4 Hz, J₂=4.8 Hz, J₃=1.4 Hz, 1H), 4.09 (tt, J₁=11.4 Hz, J₂=3.5 Hz, 1H), 3.34 (br d, J=12.6 Hz, 2H), 3.08 (br q, J=11.8 Hz, 2H), 2.02 (br d, J=12.6 Hz, 2H), 1.74 (m, 2H).

**Example 5. Preparation of the Compound of Formula XV:** To a solution of the compound of Formula XIV (150 g) in water (300 mL) was added 25% sodium hydroxide (270 mL) while maintaining temperature below 60°C. Acetic acid (34 mL) was added followed by 25-30% aqueous solution of methoxyamine hydrochloride (180 mL). The pH of the mixture was adjusted to be 3-6. The mixture was heated at 50-60°C for about 3 h. After the mixture is cooled to room temperature, 25% sodium hydroxide was added (150 mL) and the mixture was extracted with toluene (376 mL) twice. The organic layers were combined and concentrated under vacuum to give the free base (mixture of E and Z isomers in about 53:47 ratio by HPLC analysis).

**Example 6. Isomerization to I as Predominantly the E isomer:** After being azeotropically dried, the free base from Example 5 was dissolved in toluene (375 mL) and added to 5-6 N hydrochloric acid in isopropanol (300 mL). The mixture was heated at 60-70°C for 3 h, during which time the product crystallized out. The mixture was cooled to room temperature, filtered, and washed with isopropanol (300 mL). It was dried at 50-60°C to give an white solid (106.8 g, m.p.: 197°C dec., E/Z ratio: 97:3 by HPLC analysis). ¹H NMR (400 MHz, D₂O, E isomer): δ 8.61 (dd, J₁=6.1 Hz, J₂=1.2 Hz, 1H), 8.48 (dt, J₁=1.5 Hz, J₂=8.0 Hz, 1H), 8.12 (d, J=8.3 Hz, 1H), 7.90 (ddd, J₁=7.7 Hz, J₂=5.9 Hz, J₃=1.0 Hz 1H), 3.99 (s, 3H), 3.39 (m, 2H), 3.30 (tt, J₁=3.5 Hz, J₂=12.4 Hz, 1H), 2.94 (dt, J₁=2.6 Hz, J₂=13.2 Hz, 2H), 2.37 (dq, J₁=3.9 Hz, J₂=13.5 Hz, 2H), 1.93 (br d, J=14.2, 2H).

## Claims

1. A process for preparing a compound of the Formula: 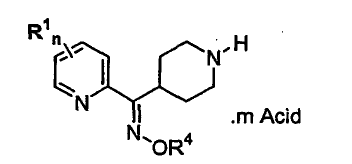 where R¹ and R⁴ are defined below, m is 1 or 2, and n is a number from 1 to 4, and wherein said compound is in its E-isomer form in at least about 90% stereochemical purity, from a compound of Formula III: 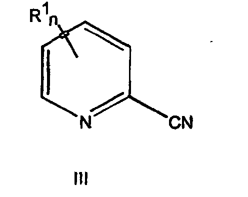 and from a compound of Formula IV: 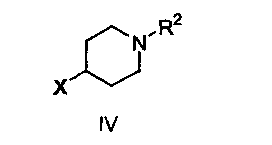 where R² is defined below and X is a halogen, said process comprising:
(a) converting the compound of Formula IV into its Grignard form of Formula IVA: 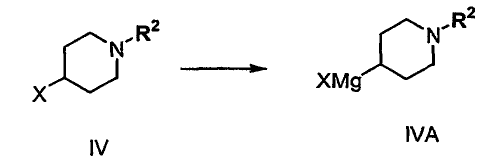 where R² is defined below and X is a halogen;
(b) reacting the compound of Formula III with the compound of Formula IVA to obtain a compound of Formula V: 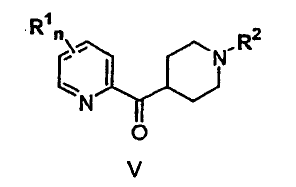
(c) reacting the compound of Formula V with a suitable alkyl chloroformate of Formula VI:
R³-OCOCl VI
where R³ is defined below, to yield a compound of Formula VII: 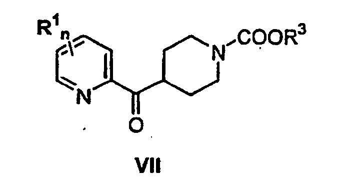
(d) converting the compound of Formula VII into its free base of Formula VIIA: 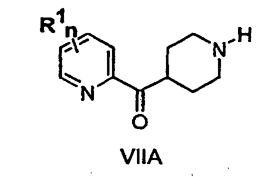
(e) Forming the acid salt (Formula VIII) from the compound of Formula VIIA: 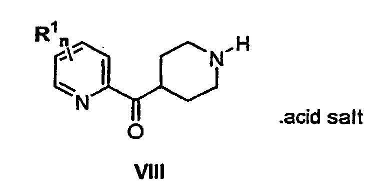
(f) reacting the compound of Formula VIII with an alkoxyamine (NH₂OR⁴) or its hydrochloride to form an oxime of Formula IX: 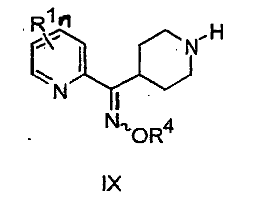 where R⁴ is defined below, and
(g) isomerizing the compound of Formula IX by treatment with a strong acid and simultaneously converting to the desired acid salt of Formula IX with an enriched E isomer, wherein the E isomer predominates over the Z-isomer by at least a 90:10 ratio,
wherein R¹, R², R³ and R⁴ may be the same or different and are independently selected from the group consisting of H, halogen, alkyl, aryl, alkoxy, aryloxy, arylalkyl (with the alkyl being the linker), alkylaryl (with the aryl being the linker), heteroalkyl, heteroaryl, alkyl-heteroaryl, heteroaralkyl, cycloalkyl and cycloalkylalkyl, wherein said alkyl, aryl, alkoxy, aryloxy, arylalkyl, alkylaryl, heteroalkyl, heteroaryl, alkyl-heteroaryl, heteroaralkyl, cycloalkyl and cycloalkylalkyl may optionally be substituted with one or more chemically-suitable substituents independently selected from the group consisting of alkyl, alkenyl, alkynyl, aryl, aralkyl, cycloalkyl, heterocyclic and halogen, and further wherein halogen refers to F, Cl, Br or I.

2. The process of claim 1, wherein X is Cl or Br, m is 2, (R¹)ₙ is H, and R² = R³= R⁴= methyl or ethyl.

3. The process of claim 2, wherein said conversion in step (a) comprises reacting said compound of Formula IV with magnesium in the presence of an initiator in a solvent, wherein said solvent is selected from the group consisting of toluene, xylene, tetrahydrofuran and mixtures thereof.

4. The process of claim 3, wherein said solvent is tetrahydrofuran.

5. The process of claim 1, wherein said initiator is sodium bis(2-methoxyethoxy)aluminum hydride or iodine.

6. The process of claim 1, wherein in step (c), said alkyl chloroformate is selected from the group consisting of methyl chloroformate, ethyl chloroformate, propyl chloroformate and benzyl chloroformate.

7. The process of claim 6, wherein said alkyl chloroformate is ethyl chloroformate, and said reaction in step (c) is conducted in a solvent selected from the group consisting of toluene, xylene, chlorobenzene, methylene chloride, ethylene chloride, ethyl acetate, isopropyl acetate, n-butyl acetate, tetrahydrofuran, methyl tetrahydrofuran and mixtures thereof.

8. The process of claim 7, wherein said solvent is toluene.

9. The process claim 1, wherein said reaction in step (d) is conducted by using acid hydrolysis or base hydrolysis.

10. The process of claim 1, wherein said acid salt in step (e) is a sulfate, hydrochloride or trifluoroacetate.

11. The process of claim 10, wherein said salt is sulfate.

12. The process of claim 11, wherein said sulfate VIII is formed in water, followed by recrystallization from a solvent, wherein said solvent for recrystallization is selected from the group consisting of water, acetonitrile, THF, ethanol, methanol, acetone and mixtures thereof.

13. The process of claim 12, wherein said solvent is acetonitrile- water mixture.

14. The process of claim 1, wherein said alkoxyamine in step (f) is methoxyamine or methoxyamine hydrochloride, said step (f) is effected in the presence of a weak acid, and said acid is acetic acid.

15. The process of claim 1, wherein said strong acid in step (g) is selected from the group consisting of HCl, HBr and H₂SO₄, and said treatment of the compound of Formula IX comprises reacting with said strong acid in a solvent at about 20 to 100°C for about 1-20 hours.

16. The process of claim 15, wherein said acid is HCl.

17. The process of claim 16, wherein said HCl is present in about 1-10 molar equivalents, with respect to the compound of Formula IX, and said solvent is selected from the group consisting of ethanol, methanol, isopropanol, n-butanol, methyl tert-butyl ether, tetrahydrofuran, heptane, hexane, toluene, acetonitrile, ethyl acetate and mixtures thereof.

18. The process of claim 17, wherein said solvent is a mixture of isopropyl alcohol and toluene.

19. A process for preparing a compound of the Formula: 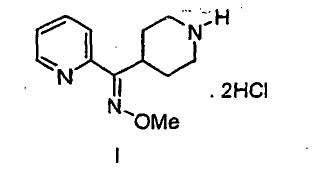 wherein said compound is in its E-isomer form in at least about 90% stereochemical purity, from a compound of Formula X: 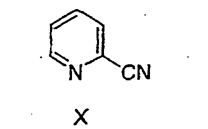 and from a compound of Formula XIA: 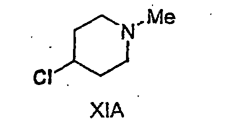 said process comprising:
(a) converting the compound of Formula XIA into its Grignard form of Formula XI: 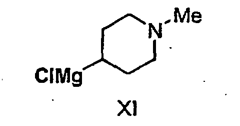
(b) reacting the compound of Formula X with the compound of Formula XI to obtain a compound of Formula XII: 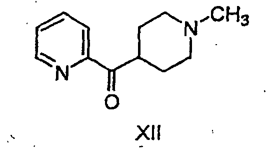
(c) reacting the compound of Formula XII with ethyl chloroformate to yield a compound of Formula XIII: 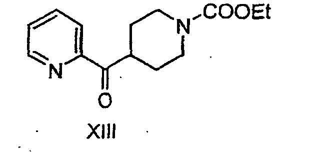
(d) converting the compound of Formula XIII into its free base of Formula XIIIA: 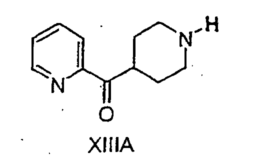
(e) forming the sulfate salt (Formula XIV) of the compound of Formula XIIIA: 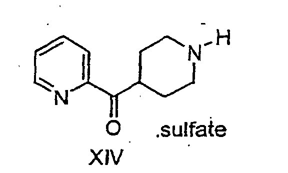
(f) reacting the compound of Formula XIV with an alkoxyamine (NH₂OR²) or its hydrochloride to form an alkyloxime of Formula XV: 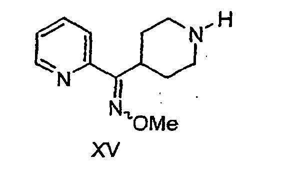 and
(g) isomerizing the compound of Formula XV by treatment with a strong acid and simultaneously converting to the desired acid salt of Formula I with an enriched E isomer, under conditions suitable to predominate the E isomer over the Z-isomer by at least a 90:10 ratio.

20. The process of claim 19, wherein said conversion in step (a) comprises reacting said compound of Formula XIA with magnesium in the presence of sodium bis(2-methoxyethoxy)aluminum hydride in a solvent selected from the group consisting of toluene, xylene, diethyl ether, tetrahydrofuran and mixtures thereof.

21. The process of claim 20, wherein said solvent is tetrahydrofuran.

22. The process of claim 19, wherein said reaction in step (c) is conducted in a solvent selected from the group consisting of toluene, xylene, chlorobenzene, methylene chloride, ethylene chloride, ethyl acetate, isopropyl acetate, n-butyl acetate, tetrahydrofuran and mixtures thereof, at about 25-100°C.

23. The process of claim 22, wherein said solvent is toluene.

24. The process claim 19, wherein said reaction in step (d) is conducted by using acid hydrolysis or base hydrolysis.

25. The process of claim 19, wherein said sulfate VIII in step (e) is formed in water, followed by recrystallization from a solvent selected from the group consisting of water, acetonitrile, THF, ethanol, methanol, acetone and mixtures thereof.

26. The process of claim 25, wherein said solvent is acetonitrile-water mixture.

27. The process of claim 19, wherein said alkoxyamine in step (f) is methoxyamine or methoxyamine hydrochloride.

28. The process of claim 19, wherein said strong acid in step (g) is selected from the group consisting of HCl, HBr and H₂SO₄, and said treatment of the compound of Formula XV comprises reacting with said strong acid in a solvent at about 20 to 100°C for about 1-20 hours, wherein said solvent is selected from the group consisting of ethanol, methanol, isopropanol, n-butanol, methyl tert-butyl ether, tetrahydrofuran, heptane, hexane, toluene, acetonitrile, ethyl acetate and mixtures thereof.

29. The process of claim 28, wherein said acid is HCl.

30. The process of claim 28, wherein said solvent is a mixture of isopropyl alcohol and toluene.

31. The process of claim 19, wherein said desired salt in step (g) is a mono acid salt.

32. The process of claim 19, wherein said desired salt in step (g) is a diacid salt.

33. The process of claim 31 or 32, wherein said acid salt is further converted to its free base.

34. A compound of the Formula:

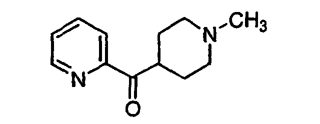

35. A compound of the Formula:

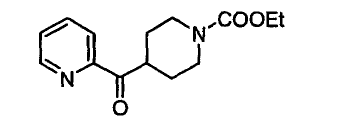

36. A compound of the formula: 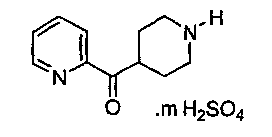 wherein m is 0, 1 or 2.

37. A compound of the formula: 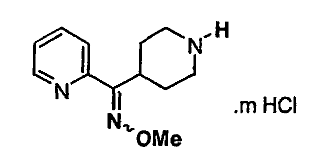 wherein m is 0, 1 or 2.

38. A process to isomerize a compound of the formula: 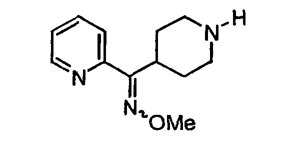 into predominantly its E isomer, wherein said E-isomer is in at least about 90% stereochemical purity, said process comprising treating said compound with a strong acid in a solvent at about 20-100°C for about 1-20 hours.

39. The process of claim 38, wherein said acid is HCl and said solvent is a mixture of isopropyl alcohol and toluene.

## Patentansprüche

1. Verfahren zur Herstellung einer Verbindung mit der Formel: 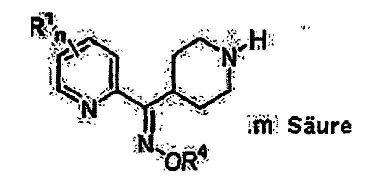 worin R¹ und R⁴ nachfolgend definiert sind, m 1 oder 2 ist und n eine Zahl von 1 bis 4 ist, und wobei die Verbindung in ihrer E-Isomerform in mindestens etwa 90 % stereochemischer Reinheit vorliegt, aus einer Verbindung mit der Formel III: 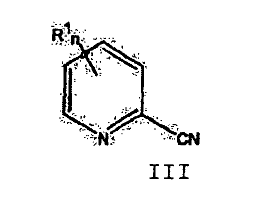 und einer Verbindung mit der Formel IV: 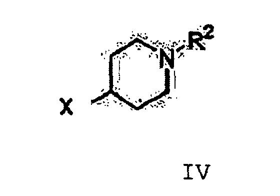 wobei R² nachfolgend definiert ist und X ein Halogen ist,
wobei das Verfahren beinhaltet:
(a) Umwandeln der Verbindung der Formel IV in ihre Grignardform der Formel IVA: 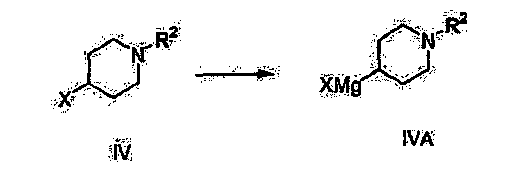 wobei R² nachfolgend definiert ist und X ein Halogen ist;
(b) Umsetzen der Verbindung der Formel III mit der Verbindung der Formel IVA, um eine Verbindung mit der Formel V zu erhalten: 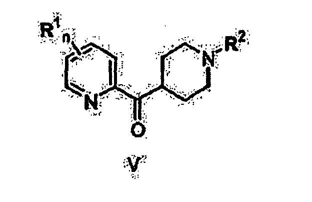
(c) Umsetzen der Verbindung der Formel V mit einem geeigneten Alkylchlorformiat mit der Formel VI:
R³-OCOCl VI
wobei R³ nachfolgend definiert ist, um eine Verbindung mit der Formel VII zu ergeben: 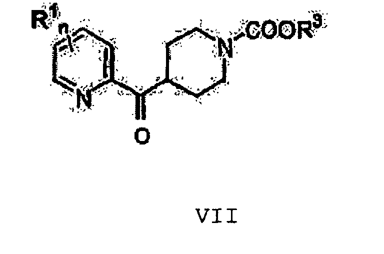
(d) Umwandeln der Verbindung mit der Formel VII in ihre freie Base mit der Formel VIIA: 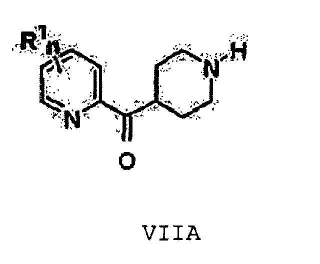
(e) Bilden des Säuresalzes (Formel VIII) aus der Verbindung mit der Formel VIIA: 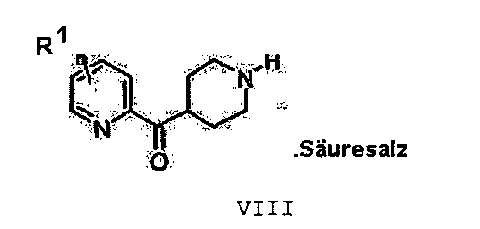
(f) Umsetzen der Verbindung mit der Formel VIII mit einem Alkoxyamin (NH₂OR⁴) oder seinem Hydrochlorid, um ein Oxim mit der Formel IX zu bilden: 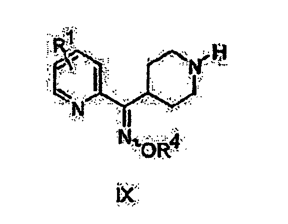 wobei R⁴ nachfolgend definiert ist, und
(g) Isomerisieren der Verbindung mit der Formel IX durch Behandlung mit einer starken Säure und gleichzeitiges Umwandeln in das gewünschte Säuresalz mit der Formel IX mit einem angereicherten E-Isomer, wobei das E-Isomer gegenüber dem Z-Isomer in mindestens einem Verhältnis von 90:10 überwiegt,
wobei R¹, R², R³ und R⁴ gleich oder unterschiedlich sein können und unabhängig ausgewählt sind aus der Gruppe bestehend aus H, Halogen, Alkyl, Aryl, Alkoxy, Aryloxy, Arylalkyl (wobei das Alkyl der Linker ist), Alkylaryl (wobei das Aryl der Linker ist), Heteroalkyl, Heteroaryl, Alkylheteroaryl, Heteroaralkyl, Cycloalkyl und Cycloalkylalkyl, wobei das Alkyl, Aryl, Alkoxy, Aryloxy, Arylalkyl, Alkylaryl, Heteroalkyl, Heteroaryl, Alkylheteroaryl, Heteroaralkyl, Cycloalkyl und Cycloalkylalkyl gegebenenfalls mit einem oder mehreren, chemisch geeigneten Substituenten substituiert sein kann, die unabhängig ausgewählt sind aus der Gruppe bestehend aus Alkyl, Alkenyl, Alkinyl, Aryl, Aralkyl, Cycloalkyl, Heterocyclen und Halogen, und wobei Halogen sich ferner auf F, Cl, Br oder I bezieht.

2. Verfahren nach Anspruch 1, bei dem X Cl oder Br ist, m 2 ist, (R¹)ₙ H ist und R² = R³ = R⁴ = Methyl oder Ethyl ist.

3. Verfahren nach Anspruch 2, bei dem die Umwandlung in Stufe (a) die Umsetzung der Verbindung mit der Formel IV mit Magnesium in Gegenwart eines Initiators in einem Lösungsmittel beinhaltet, wobei das Lösungsmittel ausgewählt ist aus der Gruppe bestehend aus Toluol, Xylol, Tetrahydrofuran und Mischungen davon.

4. Verfahren nach Anspruch 3, bei dem das Lösungsmittel Tetrahydrofuran ist.

5. Verfahren nach Anspruch 1, bei dem der Initiator Natrium-bis(2-methoxyethoxy)aluminiumhydrid oder Iod ist.

6. Verfahren nach Anspruch 1, bei dem in Stufe (c) das Alkylchlorformiat ausgewählt ist aus der Gruppe bestehend aus Methylchlorformiat, Ethylchlorformiat, Propylchlorformiat und Benzylchlorformiat.

7. Verfahren nach Anspruch 6, bei dem das Alkylchlorformiat Ethylchlorformiat ist und die Reaktion in Stufe (c) in einem Lösungsmittel ausgewählt aus der Gruppe bestehend aus Toluol, Xylol, Chlorbenzol, Methylenchlorid, Ethylenchlorid, Ethylacetat, Isopropylacetat, n-Butylacetat, Tetrahydrofuran, Methyltetrahydrofuran und Mischungen davon durchgeführt wird.

8. Verfahren nach Anspruch 7, bei dem das Lösungsmittel Toluol ist.

9. Verfahren nach Anspruch 1, bei dem die Reaktion in Stufe (d) durch Verwendung von Säurehydrolyse oder Basenhydrolyse durchgeführt wird.

10. Verfahren nach Anspruch 1, bei dem das Säuresalz in Stufe (e) ein Sulfat, Hydrochlorid oder Trifluoracetat ist.

11. Verfahren nach Anspruch 10, bei dem das Salz Sulfat ist.

12. Verfahren nach Anspruch 11, bei dem das Sulfat VIII in Wasser gebildet wird, gefolgt von Umkristallisation aus einem Lösungsmittel, wobei das Lösungsmittel für die Umkristallisation ausgewählt ist aus der Gruppe bestehend aus Wasser, Acetonitril, THF, Ethanol, Methanol, Aceton und Mischungen davon.

13. Verfahren nach Anspruch 12, bei dem das Lösungsmittel eine Acetonitril-Wasser Mischung ist.

14. Verfahren nach Anspruch 1, bei dem das Alkoxyamin in Stufe (f) Methoxyamin oder Methoxyaminhydrochlorid ist, wobei die Stufe (f) in Gegenwart einer schwachen Säure durchgeführt wird und die Säure Essigsäure ist.

15. Verfahren nach Anspruch 1, bei dem die starke Säure in Stufe (g) ausgewählt ist aus der Gruppe bestehend aus HCl, HBr und H₂SO₄, und die Behandlung der Verbindung mit der Formel IX das Umsetzen mit der starken Säure in einem Lösungsmittel bei etwa 20 bis 100°C für etwa 1 bis 20 Stunden beinhaltet.

16. Verfahren nach Anspruch 15, bei dem die Säure HCl ist.

17. Verfahren nach Anspruch 16, bei dem HCl in Bezug auf die Verbindung mit der Formel IX in etwa 1 bis 10 Moläquivalenten vorhanden ist und das Lösungsmittel ausgewählt ist aus der Gruppe bestehend aus Ethanol, Methanol, Isopropanol, n-Butanol, Methyl-tert-butylether, Tetrahydrofuran, Heptan, Hexan, Toluol, Acetonitril, Ethylacetat und Mischungen davon.

18. Verfahren nach Anspruch 17, bei dem das Lösungsmittel eine Mischung aus Isopropylalkohol und Toluol ist.

19. Verfahren zur Herstellung einer Verbindung mit der Formel: 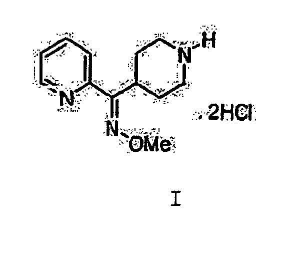 wobei die Verbindung in ihrer E-Isomerform in mindestens etwa 90 % stereochemischer Reinheit vorliegt, aus einer Verbindung mit der Formel X: 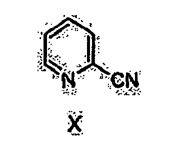 und einer Verbindung mit der Formel XIA: 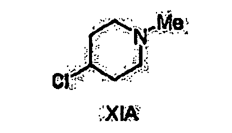 wobei das Verfahren beinhaltet:
(a) Umwandeln der Verbindung der Formel XIA in ihre Grignardform der Formel XI: 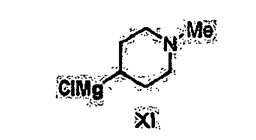
(b) Umsetzen der Verbindung der Formel X mit der Verbindung der Formel XI, um eine Verbindung mit der Formel XII zu erhalten: 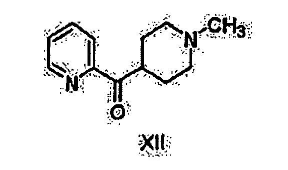
(c) Umsetzen der Verbindung mit der Formel XII mit Ethylchlorformiat, um eine Verbindung mit der Formel XIII zu ergeben: 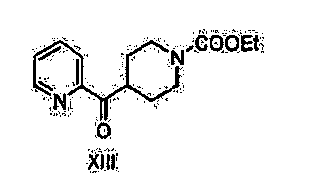
(d) Umwandeln der Verbindung mit der Formel VIII in ihre freie Base mit der Formel VIIIA: 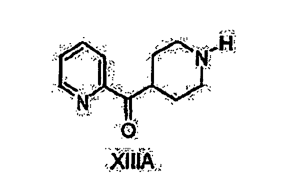
(e) Bilden des Sulfatsalzes (Formel XIV) der Verbindung mit der Formel XIIIA: 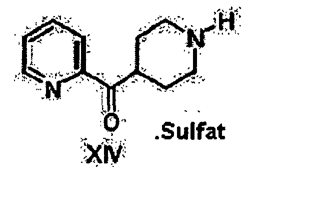
(f) Umsetzen der Verbindung mit der Formel XIV mit einem Alkoxyamin (NH₂OR²) oder seinem Hydrochlorid, um ein Alkyloxim mit der Formel XV zu bilden: 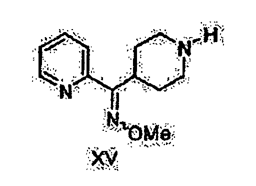 und
(g) Isomerisieren der Verbindung mit der Formel XV durch Behandlung mit einer starken Säure und gleichzeitiges Umwandeln in das gewünschte Säuresalz mit der Formel I mit einem angereicherten E-Isomer unter Bedingungen, die geeignet sind, damit das E-Isomer gegenüber dem Z-Isomer in mindestens einem Verhältnis von 90:10 überwiegt.

20. Verfahren nach Anspruch 19, bei dem die Umwandlung in Stufe (a) die Umsetzung der Verbindung mit der Formel XIA mit Magnesium in Gegenwart von Natriumbis(2-methoxyethoxy)aluminiumhydrid in einem Lösungsmittel ausgewählt aus der Gruppe bestehend aus Toluol, Xylol, Diethylether Tetrahydrofuran und Mischungen davon beinhaltet.

21. Verfahren nach Anspruch 20, bei dem das Lösungsmittel Tetrahydrofuran ist.

22. Verfahren nach Anspruch 19, bei dem die Reaktion in Stufe (c) in einem Lösungsmittel ausgewählt aus der Gruppe bestehend aus Toluol, Xylol, Chlorbenzol, Methylenchlorid, Ethylenchlorid, Ethylacetat, Isopropylacetat, n-Butylacetat, Tetrahydrofuran und Mischungen davon bei etwa 25 bis 100°C durchgeführt wird.

23. Verfahren nach Anspruch 22, bei dem das Lösungsmittel Toluol ist.

24. Verfahren nach Anspruch 19, bei dem die Reaktion in Stufe (d) durch Verwendung von Säurehydrolyse oder Basenhydrolyse durchgeführt wird.

25. Verfahren nach Anspruch 19, bei dem das Sulfat VIII in Stufe (e) in Wasser gebildet wird, gefolgt von Umkristallisation aus einem Lösungsmittel ausgewählt aus der Gruppe bestehend aus Wasser, Acetonitril, THF, Ethanol, Methanol, Aceton und Mischungen davon.

26. Verfahren nach Anspruch 25, bei dem das Lösungsmittel Acetonitril-Wasser-Mischung ist.

27. Verfahren nach Anspruch 19, bei dem das Alkoxyamin in Stufe (f) Methoxyamin oder Methoxyaminhydrochlorid ist.

28. Verfahren nach Anspruch 19, bei dem die starke Säure in Stufe (g) ausgewählt ist aus der Gruppe bestehend aus HCl, HBr und H₂SO₄, und die Behandlung der Verbindung mit der Formel XV das Umsetzen mit der starken Säure in einem Lösungsmittel bei etwa 20 bis 100°C für etwa 1 bis 20 Stunden beinhaltet, wobei das Lösungsmittel ausgewählt ist aus der Gruppe bestehend aus Ethanol, Methanol, Isopropanol, n-Butanol, Methyl-tert.-butylether, Tetrahydrofuran, Heptan, Hexan, Toluol, Acetonitril, Ethylacetat und Mischungen davon.

29. Verfahren nach Anspruch 28, bei dem die Säure HCl ist.

30. Verfahren nach Anspruch 28, bei dem das Lösungsmittel eine Mischung aus Isopropylalkohol und Toluol ist.

31. Verfahren nach Anspruch 19, bei dem das gewünschte Salz in Stufe (g) ein Monosäuresalz ist.

32. Verfahren nach Anspruch 19, bei dem das gewünschte Salz in Stufe (g) ein Disäuresalz ist.

33. Verfahren nach Anspruch 31 oder 32, bei dem das Säuresalz ferner in seine freie Base umgewandelt wird.

34. Verbindung mit der Formel:

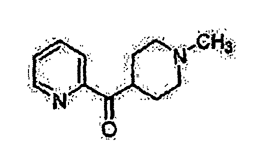

35. Verbindung mit der Formel:

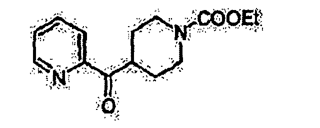

36. Verbindung mit der Formel: 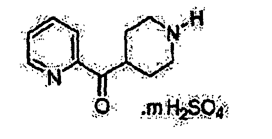 worin m 0, 1 oder 2 ist.

37. Verbindung mit der Formel: 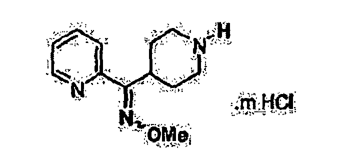 worin m 0, 1 oder 2 ist.

38. Verfahren zum Isomerisieren einer Verbindung mit der Formel: 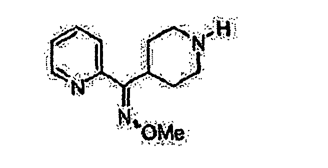 in überwiegend ihr E-Isomer, wobei das E-Isomer in mindestens etwa 90 % stereochemischer Reinheit vorliegt, wobei das Verfahren das Behandeln der Verbindung mit einer starken Säure in einem Lösungsmittel bei etwa 20 bis 100°C für etwa 1 bis 20 Stunden beinhaltet.

39. Verfahren nach Anspruch 38, bei dem die Säure HCl ist und das Lösungsmittel eine Mischung aus Isopropylalkohol und Toluol ist.

## Revendications

1. Procédé de préparation d'un composé de formule 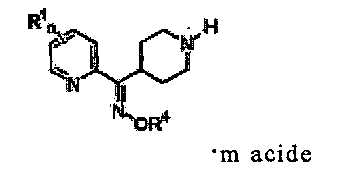 dans laquelle R¹ et R⁴ ont les significations indiquées plus loin, m vaut 1 ou 2 et n représente un nombre valant de 1 à 4,
ledit composé étant préparé sous forme de son isomère E, pur, stéréochimiquement parlant, à au moins à peu près 90 %,
à partir d'un composé de formule III : 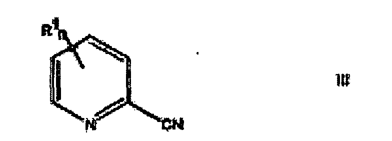 et d'un composé de formule IV : 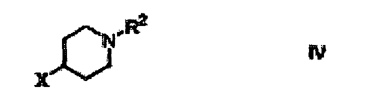 dans laquelle R² a la signification indiquée plus loin, et X représente un atome d'halogène,
lequel procédé comporte :
a) le fait de convertir le composé de formule IV en son dérivé de Grignard, de formule IVA : 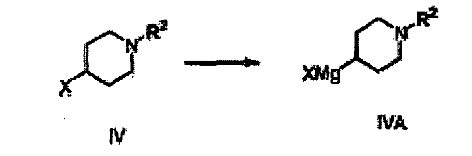 dans laquelle R² a la signification indiquée plus loin, et X représente un atome d'halogène,
b) le fait de faire réagir le composé de formule III avec ce composé de formule IVA, de manière à obtenir un composé de formule V : 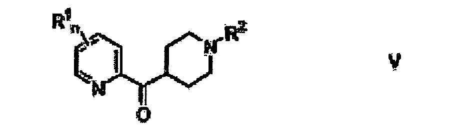
c) le fait de faire réagir ce composé de formule V avec un chloroformiate d'alkyle approprié, de formule VI :
R³-OCOCl (VI
dans laquelle R³ a la signification indiquée plus loin,
de manière à obtenir un composé de formule VII : 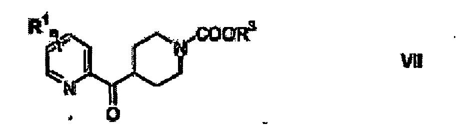
d) le fait de convertir ce composé de formule VII en la base libre correspondante, de formule VIIA : 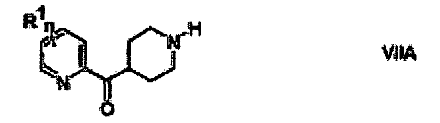
e) le fait de former un sel d'acide, de formule VIII, à partir du composé de formule VIIA : 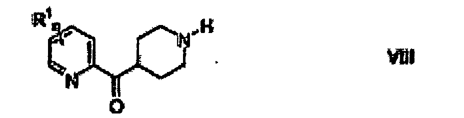 acide (sel)
f) le fait de faire réagir ce composé de formule VIII avec une alcoxylamine de formule H₂NOR⁴ ou avec un chlorhydrate d'alcoxylamine de manière à obtenir une oxime de formule IX : 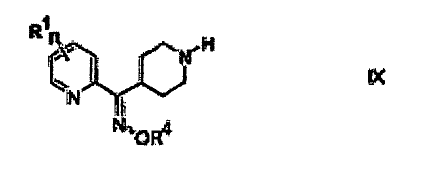 dans laquelle R⁴ a la signification indiquée plus loin,
g) et le fait d'isomériser ce composé de formule IX en le traitant avec un acide fort, ce qui permet de le convertir en même temps en le sel d'acide voulu du composé de formule IX enrichi en isomère E, où l'isomère E est prédominant en une proportion d'au moins 90/10 par rapport à l'isomère Z ;
R¹, R², R³ et R⁴ pouvant avoir des significations identiques ou différentes et représentant chacun un élément de l'ensemble formé par les atomes d'hydrogène et d'halogène et les groupes alkyle, aryle, alcoxy, aryloxy, aryl-alkyle (où le groupe alkyle constitue le raccord), alkyl-aryle (où le groupe aryle constitue le raccord), hétéroalkyle, hétéroaryle, alkyl-hétéroaryle, hétéroaryl-alkyle, cycloalkyle et cycloalkyl-alkyle, ces groupes alkyle, aryle, alcoxy, aryloxy, aryl-alkyle, alkyl-aryle, hétéroalkyle, hétéroaryle, alkyl-hétéroaryle, hétéroaryl-alkyle, cycloalkyle et cycloalkyl-alkyle pouvant en option porter un ou plusieurs substituants chimiquement appropriés, choisis indépendamment dans l'ensemble constitué par les groupes alkyle, alcényle, alcynyle, aryle, aralkyle, cycloalkyle et hétérocyclyle et les atomes d'halogène, le terme "halogène" signifiant fluor, chlore, brome ou iode.

2. Procédé conforme à la revendication 1, dans lequel X représente un atome de chlore ou de brome, m vaut 2, (R¹)ₙ représente des atomes d'hydrogène, et R², R³ et R⁴ représentent chacun un groupe méthyle ou éthyle.

3. Procédé conforme à la revendication 2, dans lequel ladite conversion de l'étape (a) consiste à faire réagir ledit composé de formule IV avec du magnésium, en présence d'un amorceur et dans un solvant, lequel solvant est choisi dans l'ensemble constitué par le toluène, le xylène et le tétrahydrofurane, ainsi que leurs mélanges.

4. Procédé conforme à la revendication 3, dans lequel ledit solvant est du tétrahydrofurane.

5. Procédé conforme à la revendication 1, dans lequel ledit amorceur est de l'hydrure de sodium et de bis(2-méthoxy-éthoxy)aluminium ou de l'iode.

6. Procédé conforme à la revendication 1, dans lequel ledit chloroformiate d'alkyle employé dans l'étape (c) est choisi dans l'ensemble formé par le chloroformiate de méthyle, le chloroformiate d'éthyle, le chloroformiate de propyle et le chloroformiate de benzyle.

7. Procédé conforme à la revendication 6, dans lequel ledit chloroformiate d'alkyle est du chloroformiate d'éthyle, et la réaction de l'étape (c) est effectuée dans un solvant choisi dans l'ensemble formé par les toluène, xylène, chlorobenzène, chlorure de méthylène, chlorure d'éthylène, acétate d'éthyle, acétate d'isopropyle, acétate de n-butyle, tétrahydrofurane et méthyl-tétrahydrofurane, ainsi que leurs mélanges.

8. Procédé conforme à la revendication 7, dans lequel ledit solvant est du toluène.

9. Procédé conforme à la revendication 1, dans lequel ladite réaction de l'étape (d) est réalisée par hydrolyse en milieu acide ou basique.

10. Procédé conforme à la revendication 1, dans lequel ledit sel d'acide formé dans l'étape (e) est un sulfate, un chlorhydrate ou un trifluoroacétate.

11. Procédé conforme à la revendication 10, dans lequel ledit sel est un sulfate.

12. Procédé conforme à la revendication 11, dans lequel ledit sulfate de formule VIII est formé dans de l'eau et récupéré ensuite par recristallisation dans un solvant, lequel solvant de recristallisation est choisi dans l'ensemble constitué par l'eau, l'acétonitrile, le tétrahydrofurane, l'éthanol, le méthanol et l'acétone, ainsi que leurs mélanges.

13. Procédé conforme à la revendication 12, dans lequel ledit solvant est un mélange d'acétonitrile et d'eau.

14. Procédé conforme à la revendication 1, dans lequel ladite alcoxylamine employée dans l'étape (f) est de la méthoxylamine ou du chlorhydrate de méthoxylamine, ledit l'étape (f) est conduit en présence d'un acide faible, et ledit acide est de l'acide acétique.

15. Procédé conforme à la revendication 1, dans lequel ledit acide fort utilisé dans l'étape (g) est choisi parmi les acides chlorhydrique, bromhydrique et sulfurique, et ledit traitement du composé de formule IX consiste à le faire réagir avec ledit acide fort, dans un solvant, à une température d'à peu près 20 à 100 °C et pendant à peu près 1 à 20 heures.

16. Procédé conforme à la revendication 15, dans lequel ledit acide est de l'acide chlorhydrique.

17. Procédé conforme à la revendication 16, dans lequel on utilise dudit acide chlorhydrique à raison d'à peu près 1 à 10 équivalents molaires par rapport au composé de formule IX, et ledit solvant est choisi dans l'ensemble formé par les éthanol, méthanol, isopropanol, n-butanol, méthyl-tertiobutyl-éther, tétrahydrofurane, heptane, hexane, toluène, acétonitrile et acétate d'éthyle, ainsi que leurs mélanges.

18. Procédé conforme à la revendication 17, dans lequel ledit solvant est un mélange d'isopropanol et de toluène.

19. Procédé de préparation d'un composé de formule 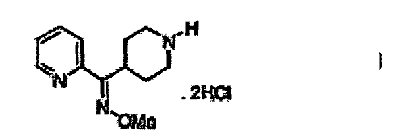 ledit composé étant préparé sous forme de son isomère E, pur, stéréochimiquement parlant, à au moins à peu près 90 %,
à partir d'un composé de formule X : 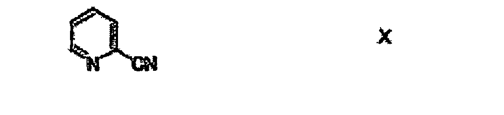 et d'un composé de formule XIA : 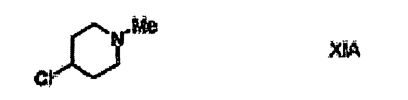 lequel procédé comporte :
a) le fait de convertir le composé de formule XIA en son dérivé de Grignard, de formule XI : 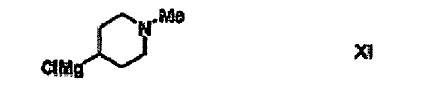
b) le fait de faire réagir le composé de formule X avec ce composé de formule XI, de manière à obtenir un composé de formule XII : 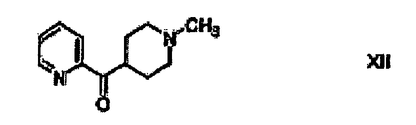
c) le fait de faire réagir ce composé de formule XII avec du chloroformiate d'éthyle, de manière à obtenir un composé de formule XIII : 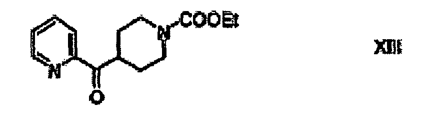
d) le fait de convertir ce composé de formule XIII en la base libre correspondante, de formule XIIIA : 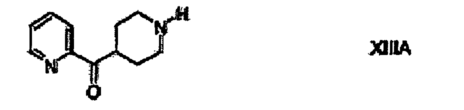
e) le fait de former le sulfate (formule XIV) de ce composé de formule XIIIA : 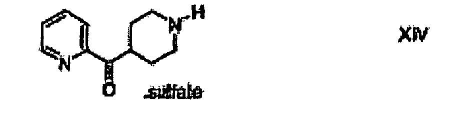
f) le fait de faire réagir ce composé de formule XIV avec une alcoxylamine de formule NH₂OR² ou un chlorhydrate d'alcoxylamine, de manière à ob-tenir une alkyl-oxime de formule XV : 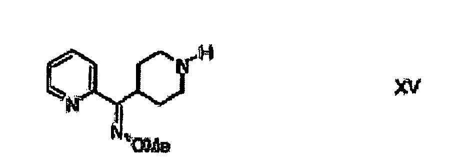
g) et le fait d'isomériser ce composé de formule XV en le traitant avec un acide fort, ce qui permet de le convertir en même temps en le sel d'acide voulu du composé de formule I enrichi en isomère E, dans des conditions appropriées pour que l'isomère E soit prédominant en une proportion d'au moins 90/10 par rapport à l'isomère Z.

20. Procédé conforme à la revendication 19, dans lequel ladite conversion de l'étape (a) consiste à faire réagir ledit composé de formule XIA avec du magnésium, en présence d'hydrure de sodium et de bis(2-méthoxyéthoxy)aluminium et dans un solvant choisi dans l'ensemble constitué par le toluène, le xylène, l'éther diéthylique et le tétrahydrofurane, ainsi que leurs mélanges.

21. Procédé conforme à la revendication 20, dans lequel ledit solvant est du tétrahydrofurane.

22. Procédé conforme à la revendication 19, dans lequel la réaction de l'étape (c) est réalisée à une température d'à peu près 25 à 100 °C, dans un solvant choisi dans l'ensemble que forment les toluène, xylène, chlorobenzène, chlorure de méthylène, chlorure d'éthylène, acétate d'éthyle, acétate d'isopropyle, acétate de n-butyle et tétrahydrofurane, ainsi que leurs mélanges.

23. Procédé conforme à la revendication 22, dans lequel ledit solvant est du toluène.

24. Procédé conforme à la revendication 19, dans lequel la réaction de l'étape (d) est réalisée par hydrolyse en milieu acide ou basique.

25. Procédé conforme à la revendication 19, dans lequel, lors de l'étape (e), ledit sulfate de formule VIII est formé dans de l'eau et récupéré ensuite par recristallisation dans un solvant choisi dans l'ensemble formé par l'eau, l'acétonitrile, le tétrahydrofurane, l'éthanol, le méthanol et l'acétone, ainsi que leurs mélanges.

26. Procédé conforme à la revendication 25, dans lequel ledit solvant est un mélange d'acétonitrile et d'eau.

27. Procédé conforme à la revendication 19, dans lequel ladite alcoxylamine employée dans l'étape (f) est de la méthoxylamine ou du chlorhydrate de méthoxylamine.

28. Procédé conforme à la revendication 19, dans lequel ledit acide fort utilisé dans l'étape (g) est choisi parmi les acides chlorhydrique, bromhydrique et sulfurique, et ledit traitement du composé de formule XV consiste à le faire réagir avec ledit acide fort, dans un solvant, à une température d'à peu près 20 à 100 °C et pendant à peu près 1 à 20 heures, ledit solvant étant choisi dans l'ensemble formé par les éthanol, méthanol, isopropanol, n-butanol, méthyl-tertiobutyl-éther, tétrahydrofurane, heptane, hexane, toluène, acétonitrile et acétate d'éthyle, ainsi que leurs mélanges.

29. Procédé conforme à la revendication 28, dans lequel ledit acide est de l'acide chlorhydrique.

30. Procédé conforme à la revendication 28, dans lequel ledit solvant est un mélange d'isopropanol et de toluène.

31. Procédé conforme à la revendication 19, dans lequel ledit sel d'acide voulu formé dans l'étape (g) est un mono-sel d'acide.

32. Procédé conforme à la revendication 19, dans lequel ledit sel d'acide voulu formé dans l'étape (g) est un di-sel d'acide.

33. Procédé conforme à la revendication 31 ou 32, dans lequel ledit sel d'acide est ensuite convertie en la base libre correspondante.

34. Composé de formule :

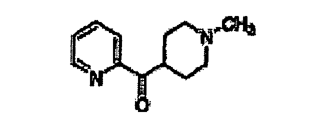

35. Composé de formule :

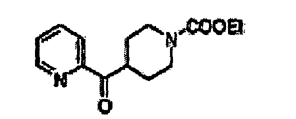

36. Composé de formule : 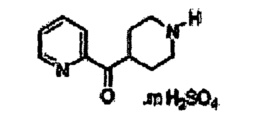 dans laquelle m vaut 0, 1 ou 2.

37. Composé de formule : 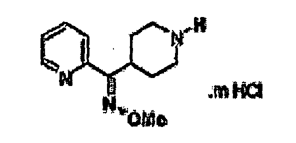 dans laquelle m vaut 0, 1 ou 2.

38. Procédé permettant d'isomériser un composé de formule : 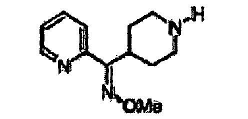 pour en obtenir de manière prédominante l'isomère E, pur, stéréochimiquement parlant, à au moins à peu près 90 %, lequel procédé comporte le fait de traiter ledit composé avec un acide fort, dans un solvant, à une température d'à peu près 20 à 100 °C et pendant à peu près 1 à 20 heures.

39. Procédé conforme à la revendication 38, dans lequel ledit acide est de l'acide chlorhydrique et ledit solvant est un mélange d'isopropanol et de toluène.
